# EUROPEAN PATENT APPLICATION

(11) **EP 0 994 095 A1**
(43) Date of publication of application: **19.04.2000**
(21) Application number: 97949223.8
(22) Date of filing: 24.12.1997
(51) Int. Cl.: C07C 59/64, C07C 69/734, C07C 229/36, C07C 255/16, C07C 255/24, C07C 323/51, C07C 309/24, C07C 323/64, C07D 209/18, C07D 213/06, C07D 239/88, C07D 261/20, C07D 263/30, C07D 413/12, C07D 401/12, C07D 401/06, C07D 409/12, C07D 413/06, C07D 417/12, C07D 215/22, C07F 9/38, A61K 31/42, A61K 31/47, A61K 31/405, A61K 31/425

(54) **PROPIONIC ACID DERIVATIVES**

(30) Priority: 24.12.1996 JP 35573096; 06.03.1997 JP 6916897
(71) Applicant: NIPPON CHEMIPHAR CO., LTD., Tokyo 101-8678 (JP)
(72) Inventor: NOMURA, Yutaka, Chiba 278-0011 (JP); SAKUMA, Shogo, Saitama 342-0055 (JP); MASUI, Seiichiro, Saitama 362-0072 (JP)
(74) Representative: Kraus, Walter, Dr.
(86) International application number: JP9704777
(87) International publication number: WO9828254

(57) **Abstract**

A propionic acid derivative having the following formula (II) and its salt: 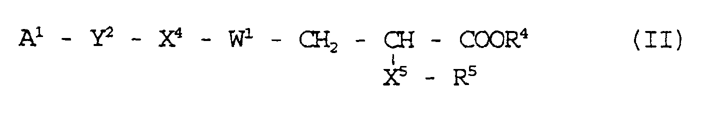 [wherein A¹ is an aryl or heterocyclic group which may have a substituent; Y² is an alkylene chain having 1 to 5 carbon atoms; X⁴ is a single bond, an oxygen atom, or a sulfur atom; W¹ is a naphthalene ring, a quinoline ring, an indole ring, a benzisoxazole ring, or benzo[b]thiophene ring, all of which may have a substituent; R⁴ is a hydrogen atom or an alkyl group having 1 to 8 carbon atoms; X⁵ is an oxygen atom or a sulfur atom; R⁵ is an alkyl group having 1 to 8 carbon atoms, an aralkyl group, or an aryl group, all of which may have a substituent] have a hypoglycemic effect and hypolipidemic activity.

## Description

### FIELD OF THE INVENTION

This invention relates to propionic acid derivatives having hypoglycemic effect.

### BACKGROUND OF THE INVENTION

Heretofore, treatment of diabetes has been performed using a parenteral injection such as an insulin preparation or an oral antidiabetic such as a biguanide compound (e.g., metformin hydrochloride) or a sulfonylurea compound (e.g., tolbutamide). The insulin preparation, however, has a disadvantageous problem in its troublesome procedure which is inherent to parenteral injection. The orally administrable biguanide compound brings about unfavorable lactic acidosis, and the sulfonylurea compound gives such side-effect as grave hypoglycemia.

Recently, attention has been paid to 5-(substituted benzyl)thiazoline-2,4-dione derivatives such as the below-illustrated troglitazone (European Patent No. 139,421) and pioglitazone hydrochloride (European Patent No. 193,256) which have no noticeable defects such as above and show new pharmacological mechanism such as curing of defective insulin sensitivity (insulin resistance). It has been reported that troglitazone shows hypoglycemic and hypolipidemic effects, cures damaged insulin-receptor function, and gives influence to glucose transporter and glucokinase, so that defective insulin sensitivity can be cured.

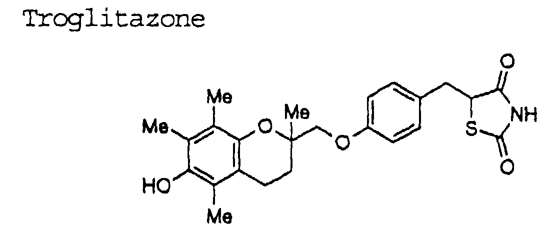

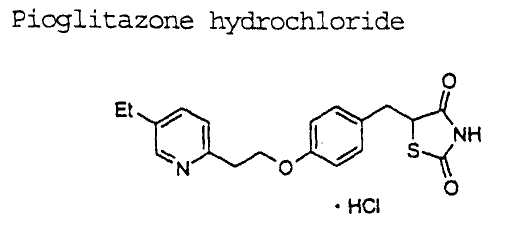

Further, it has been reported that carboxylic acid derivatives other than the above-mentioned 5-(substituted benzyl)thiazoline-2,4-dione derivatives, such as the below-illustrated derivatives, can cure defective insulin activity.

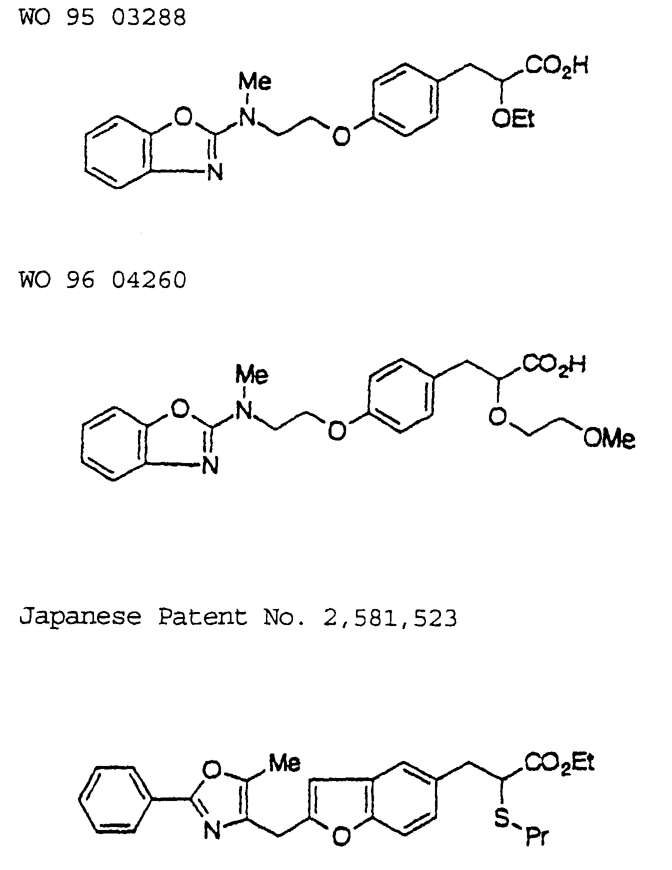

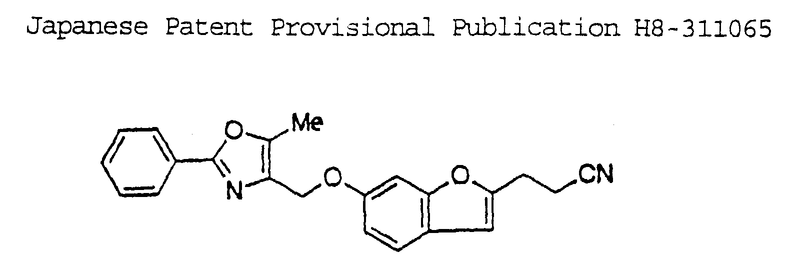

The present invention has an object to provide compounds which have a novel chemical structure and show reliable hypoglycemic effect and further show hypolipidemic effect.

### SUMMARY OF THE INVENTION

The present inventors have studied new compounds showing hypoglycemic effect and, as a result, have discovered that the compounds of the following formula (I) and their salts have excellent hypoglycemic activity and hypolipidemic effect: 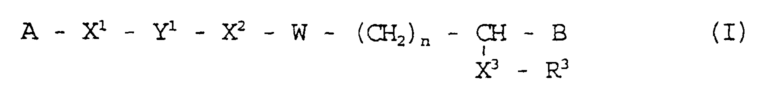 [wherein A represents an aryl or heterocyclic group which may have a substituent; X¹ represents a single bond, an oxygen atom, a sulfur atom, or NR¹ (R¹ is a hydrogen atom, an alkyl group having 1 to 8 carbon atoms, or an aralkyl group); Y¹ represents an alkylene chain having 1 to 8 carbon atoms and possibly having a substituent; X² represents a single bond, an oxygen atom, a sulfur atom, or NR² (R² is a hydrogen atom, an alkyl group having 1 to 8 carbon atoms, or an aralkyl group); W represents a naphthalene ring which may have a substituent, or a bicyclic group comprising a benzene ring which is fused with a heterocycle which contains at least one nitrogen or sulfur atom as ring member atoms and possibly having a substituent; B represents a carboxyl group, a cyano group, an alkoxycarbonyl group having 2 to 9 carbon atoms, an aralkyloxyrcarbonyl group, an aryloxycarbonyl, a sulfonic acid group, a phosphonic acid group, or a tetrazolyl group; X³ represents an oxygen atom or a sulfur atom; R³ represents an alkyl group having 1 to 8 carbon atoms, an aralkyl group, or an aryl group, all of which may have a substituent; and n is an integer of 1 to 4].

Accordingly, the present invention resides in the compounds of the formula (I) and their salts.

The invention further resides in propionic acid derivatives having the following formula (II) and their salts: 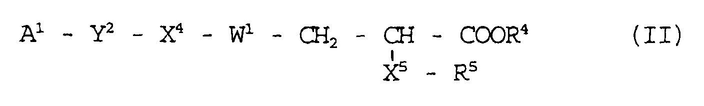 [wherein A¹ represents an aryl or heterocyclic group which may have a substituent; Y² represents an alkylene chain having 1 to 5 carbon atoms; X⁴ represents a single bond, an oxygen atom, or a sulfur atom; W¹ represents a naphthalene ring, a quinoline ring, an indole ring, a benzisoxazole ring, or benzo[b]thiophene ring, all of which may have a substituent; R⁴ represents a hydrogen atom or an alkyl group having 1 to 8 carbon atoms; X⁵ represents an oxygen atom or a sulfur atom; R⁵ represents an alkyl group having 1 to 8 carbon atoms, an aralkyl group, or an aryl group, all of which may have a substituent].

### PREFERRED EMBODIMENTS OF THE INVENTION

More detailed description of the symbols in the formula (I) are given below.
"A" typically is an aryl group such as phenyl or naphthyl, or a heterocyclic group such as pyridyl, thiazolyl, oxazolyl, chromanyl, thienyl, furyl, pyrrolyl, benzoxazolyl, morpholinyl, indolyl, benzimidazolyl, benzthiazolyl, piperidinyl, or pyrimidinyl. Preferred are phenyl and oxazolyl. These groups may have a substituent.
"X¹" is a single bond, an oxygen atom, a sulfur atom, or NR¹ (R¹ typically is a hydrogen atom, an alkyl group having 1 to 8 carbon atoms, such as methyl, ethyl, propyl or isopropyl, or an aralkyl group such as benzyl or phenethyl). Preferred is a single bond.
"Y¹" is an alkylene chain having 1 to 8 carbon atoms, preferably 1 to 5 carbon atoms, more preferably 1 to 3 carbon atoms, which may have a substituent such as oxo, a hydroxyl group, an alkyl group having 1 to 8 carbon atoms (e.g., methyl, ethyl, propyl or isopropyl) and phenyl.
"X²" is a single bond, an oxygen atom, a sulfur atom, or NR² (R² typically is the atom or group which is described hereinbefore for R¹). Preferred are a single bond and an oxygen atom.
"W" is a naphthalene ring which may have a substituent, or a bicyclic group comprising a benzene ring which is fused with a heterocycle which contains at least one nitrogen or sulfur atom as ring member atoms and possibly having a substituent. The bicyclic group typically is a quinoline ring, an indole ring, a benzisoxazole ring, benzo[b]thiophene ring, an isoquinoline ring, a benzothiazole ring, a benzoxazole ring or a benzimidazole ring. Preferred are a naphthalene ring, a quinoline ring, an indole ring, a benzisoxazole ring and a benzo[b]thiophene ring, all of which may have a substituent. Most preferred are a quinoline ring, an indole ring and a benzisoxazole ring, all of which may have a substituent.
"B" is an acidic group such as a carboxyl group, a cyano group, an alkoxycarbonyl group having 2 to 9 carbon atoms (e.g., methoxycarbonyl, ethoxycarbonyl, propoxycarbonyl, or isopropoxycarbonyl), an aralkyloxycarbonyl (e.g., benzyloxycarbonyl), an aryloxycarbonyl (e.g., phenoxycarbonyl), a sulfonic acid group, a phosphonic acid group, or a tetrazolyl group. Preferred are a carboxyl group and an alkoxycarbonyl group having 2 to 7 carbon atoms, such as methoxycarbonyl, ethoxycarbonyl, propoxycarbonyl or isopropoxycarbonyl.
"X³" is an oxygen atom or a sulfur atom. The oxygen atom is preferred.
"R³" is an alkyl group having 1 to 8 carbon atoms (e.g., methyl, ethyl, propyl or isopropyl), an aralkyl group (e.g., benzyl or phenethyl), or an aryl group (e.g., phenyl), all of which may have a substituent.
"n" is an integer of 1 to 4. Preferred is 1.

The substituent which may be attached to the aryl group or heterocyclic group represented by the above-mentioned "A" or the naphthalene ring or bicyclic ring represented by "W" can be an alkyl group having 1 to 6 carbon atom (e.g., methyl, ethyl, propyl, or isopropyl), an alkoxy group having 1 to 6 carbon atoms (e.g., methoxy or ethoxy), a halogen atom (e.g., chlorine or fluorine), an alkyl group having 1 to 6 carbon atom and being substituted with 1 to 3 halogen atoms (e.g., 2-chloroethyl or trifluoromethyl), an alkoxy group having 1 to 6 carbon atoms and being substituted with 1 to 3 halogen atoms (e.g., 2-chloroethoxy), a hydroxyl group, a nitro group, or an amino group (e.g., amino(NH₂), methylamino, ethylamino, dimethylamino, or diethylamino).

In addition, the substituent which may be attached to the aryl group or heterocyclic group represented by the above-mentioned "A" may be a phenyl group, a thienyl group, a furyl group, a thiazolyl group, or a pyridyl group. These substituent groups can have a substituent, which typically is an alkyl group having 1 to 6 carbon atoms (e.g., methyl, ethyl, propyl, or isopropyl), an alkoxy group having 1 to 6 carbon atoms (e.g., methoxy or ethoxy), a halogen atom (e.g, chlorine or fluorine), a hydroxyl group.

The substituent which may be attached to "R³" can be a hydroxyl group, an alkoxy group having 1 to 6 carbon atoms (e.g., methoxy or ethoxy), an aralkyloxy group (e.g., benzyloxy), or an axyloxy group (e.g., phenoxy).

The groups of the following formulas (III) and (IV):

A - X¹ - Y¹ - X² - (III)

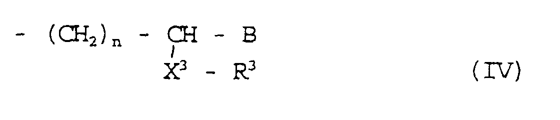

are preferably attached to the group of "W" in the following manner.

In the case that "W" is a naphthalene ring, each of the groups of the formulas (III) and (IV) are attached to each of the benzene rings of the naphthalene ring.

In the case that "W" is the bicyclic ring comprising a benzene ring which is fused with a heterocycle containing at least one nitrogen or sulfur atom as the ring member atom, the group of the formula (III) is attached to the benzene ring and the group of the formula (IV) is attached to the heterocycle. However, in the case that "W" is a benzisoxazole ring, the group of the formula (IV) is attached to the benzene ring and the group of the formula (III) is attached to the isoxazole ring.

More detailed description of the symbols in the formula (II) are given below.
"A¹" typically is one of the groups described above for "A".
"Y²" is an alkylene chain having 1 to 5 carbon atoms, and preferably is an alkylene chain having 1 to 3 carbon atom.
"X⁴" is a single bond, an oxygen atom, or a sulfur atom. Preferred are a single bond and an oxygen atom.
"W¹" is a naphthalene ring, a quinoline ring, an indole ring, a benzisoxazole ring, or benzo[b]thiophene ring, and these rings may have a substituent. Preferred are a quinoline ring, an indole ring, and a benzisoxazole ring.
"R⁴" is a hydrogen atom or an alkyl group having 1 to 8 carbon atoms (e.g., methyl, ethyl, propyl, or isopropyl).
"X⁵" is an oxygen atom or a sulfur atom. An oxygen atom is preferred.
"R⁵" typically is one of the groups described above for "R³" of the formula (I).

The substituent which may be attached to the aryl group or heterocyclic group represented by "A¹" and the substituent which may be attached to the naphthalene ring or bicyclic ring having a heterocyclic ring represented by "W¹" can be one of the substituent groups for the aryl group or heterocyclic group represented by "A" of the formula (I) and one of the substituent groups for the naphthalene ring or bicyclic ring represented by "W" of the formula (I), respectively.

The groups of the following formulas (V) and (VI):

A¹ - Y² - X⁴ - (V)

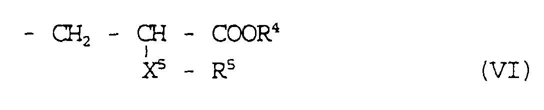

are preferably attached to the group of "W¹" in the following manner.

In the case chat. "W¹" is a naphthalene ring, each of the groups of the formulas (V) and (VI) are attached to each of the benzene rings of the naphthalene ring.

In the case that "W¹" is a quinoline ring, an indole ring, or a benzo[b]thiophene ring, the group of the formula (V) is attached to the benzene ring and the group of the formula (VI) is attached to the heterocycle. In the case that "W¹" is a benzisoxazole ring, the group of the formula (VI) is attached to the benzene ring and the group of the formula (V) is attached to the isoxazole ring.

Further, the group of the formula (VI) is preferably attached to the naphthalene ring at 2-position, quinoline ring at 2- or 3-position, indole or benzo[b]thiophene ring at 2-position, and benzisoxazole at 6-position. The group of the formula (V) is preferably attached to the naphthalene or quinoline ring at 6- or 7-position, indole or benzo[b]thiophene ring at 5- or 6-position, and benzisoxazole ring at 3-position.

The compounds of the invention [namely, the compounds represented by the formula (I) and the propionic acid derivatives represented by the formula (II)] can be employed in the form of a pharmacologically acceptable salt. The salt can be an acidic salt with hydrochloric acid or acetic acid, or a basic salt with an alkali metal (e.g., sodium or potassium).

The carbon atom to which "B" is attached in the formula (I) and the carbon atom to which COOR⁴ is attached in the formula (II) are both asymmetric carbon atoms. Therefore, the compounds of the invention can be present in the form of an optical isomer or a racemic mixture. These optical isomers and racemic mixture are included in the compounds of the invention.

The propionic acid derivatives of the formula (II) can be prepared, for instance, according to the following reaction scheme:

### Synthesis Process 1)

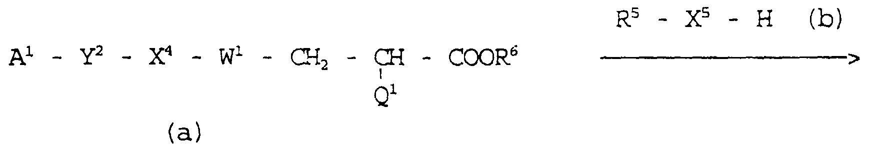

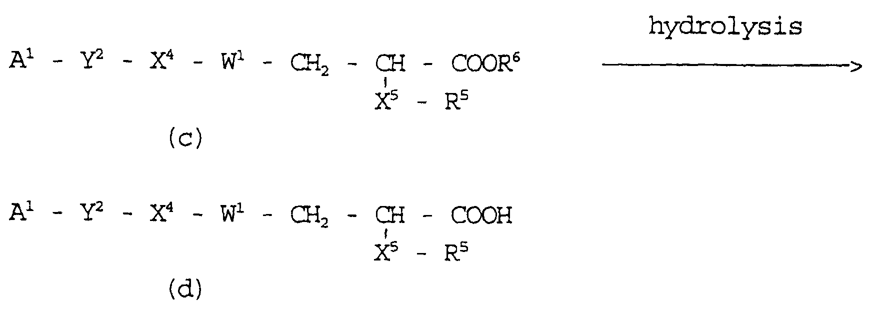

In the above-mentioned formulas, Q¹ represents an eliminable group such as a halogen atom (e.g., chlorine or bromine) or tosyloxy group, R⁶ is an alkyl group having 1 to 8 carbon atoms (e.g., methyl, ethyl, propyl, or isopropyl), and each of A¹, Y², X⁴, W¹, X⁵ and R⁵ has the meaning mentioned hereinbefore.

The propionic acid ester of the invention which is represented by the formula (c) can be obtained by reaction between an propionic acid ester of the formula (a) and a compound of the formula (b). The reaction can be performed in an inert solvent (e.g., benzene or tetrahydrofuran (THF)) in the presence of a base (e.g., NaH or LDA).

The propionic acid ester of the invention which is represented by the formula (d) can be obtained by subjecting the propionic acid ester of the formula (c) to known hydrolysis.

The propionic acid ester of the formula (a) can be prepared by the process (according to Meerwein reaction) which is described in WO 96 12719.

### Synthesis Process 2)

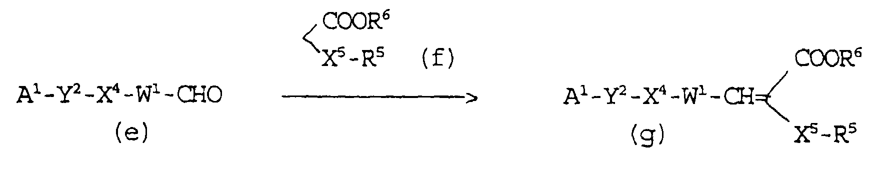

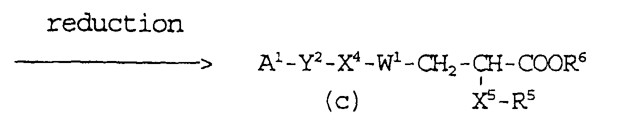

In the above-mentioned formulas, each of A¹, Y², X⁴, W¹, X⁵, R⁵ and R⁶ has the meaning mentioned hereinbefore.

The acrylic acid ester of the formula (g) also can be obtained according to the following process:

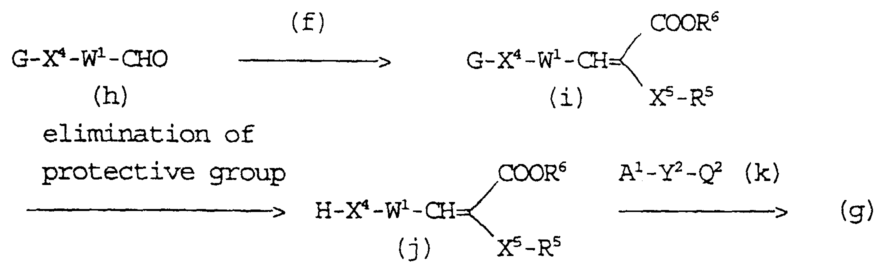

In the above-mentioned formulas, G is a protective group for X⁴, Q² is an eliminable group such as that of Q¹, and each of A¹, Y², X⁴, W¹, X⁵, R⁵ and R⁶ has the meaning mentioned hereinbefore.

### Synthesis Process 3)

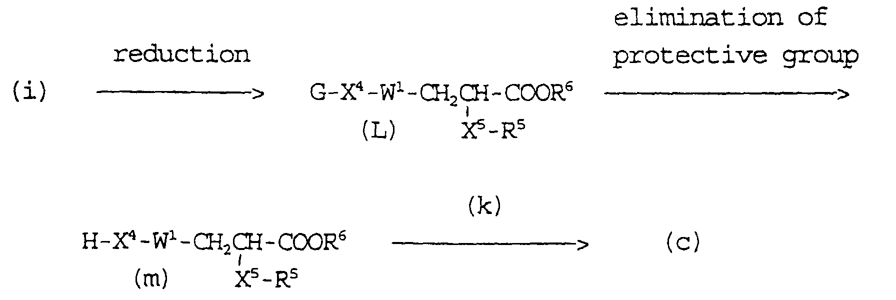

In the above-mentioned formulas, G, X⁴, W¹, X⁵, R⁵ and R⁶ has the meaning mentioned hereinbefore.

### Synthesis Process 4)

(1) Reaction of α-diazoarylpropionic acid ester with rhodium(II) acetate in alcohol (Tetrahedron Letters, vol.35, No.19, p.3139-3142 (1994))
(2) Reaction of α-hydroxyarylpropionic acid ester with an alkyl halide (WO 94 01420).

The compounds of the formula (I) which are not included in the propionic acid esters of the formula (II) can be prepared in an analogous manner.

Representative examples of the propionic acid esters of the formula (II) according to the invention are set forth in Tables 1 to 5.

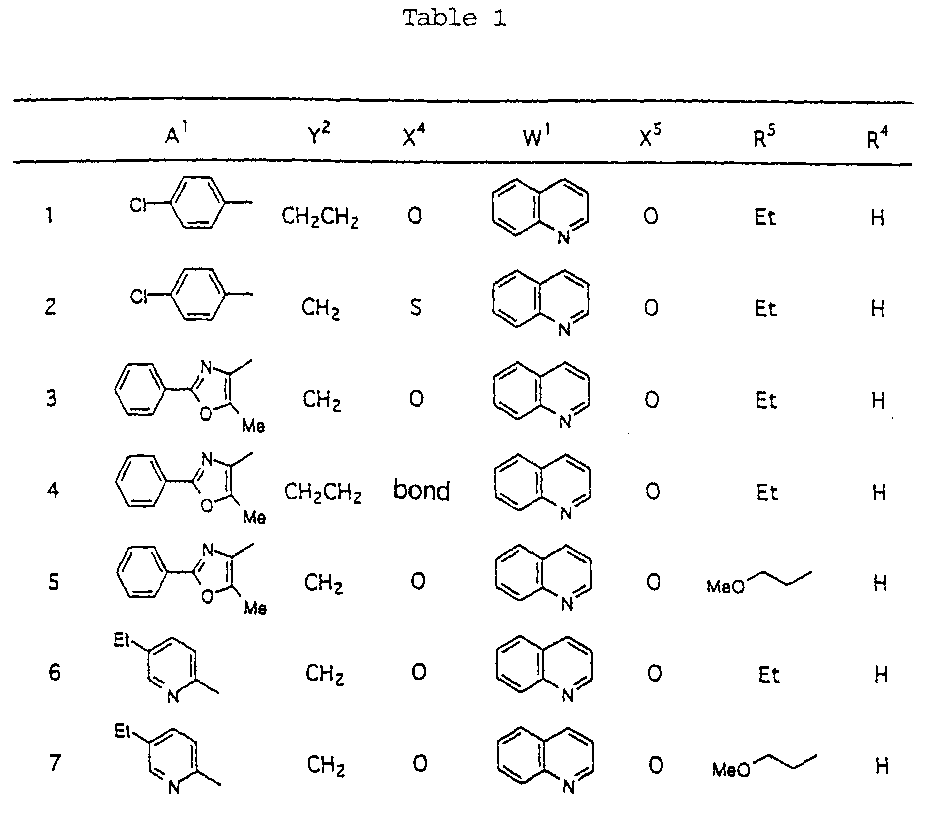

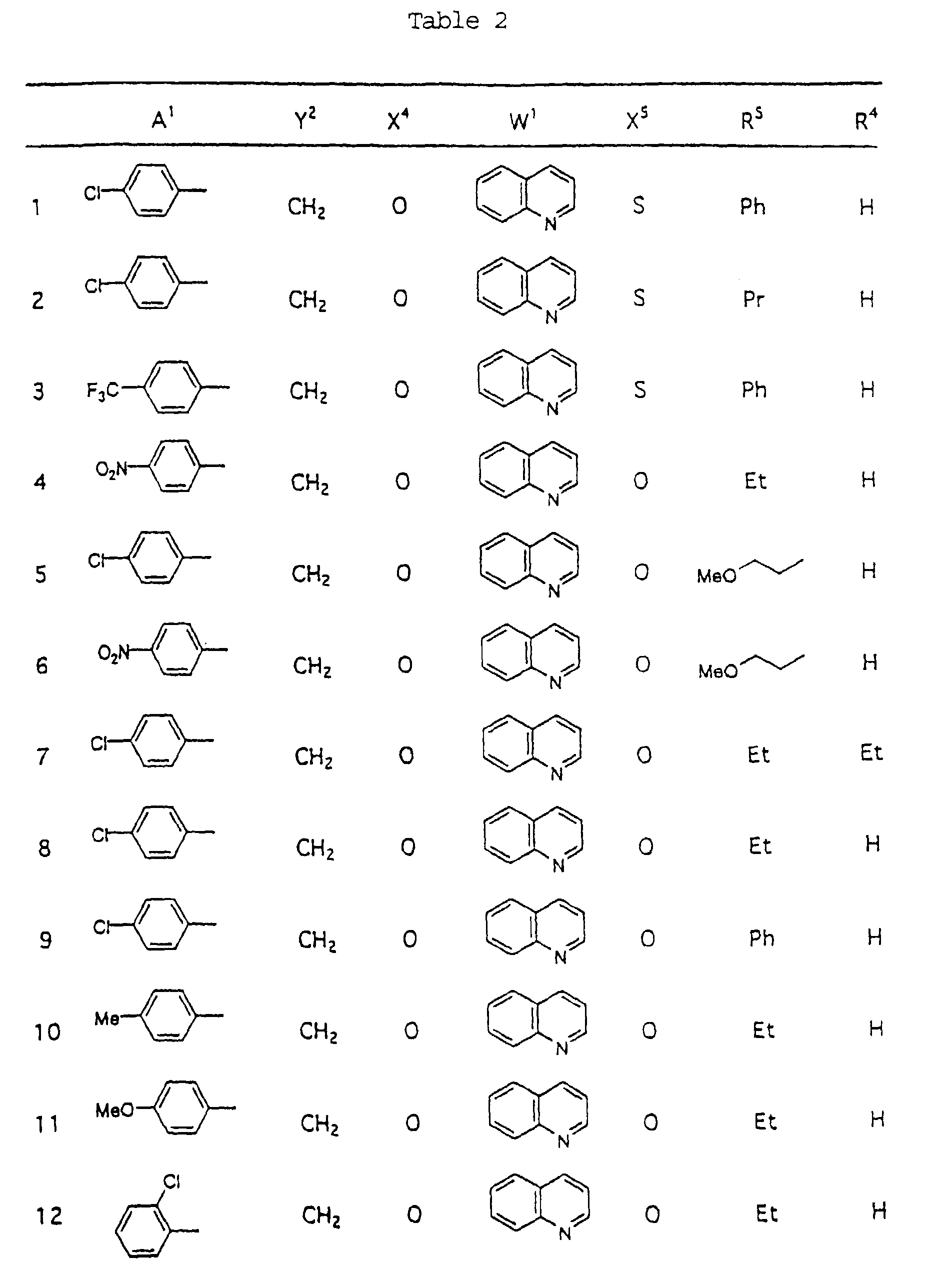

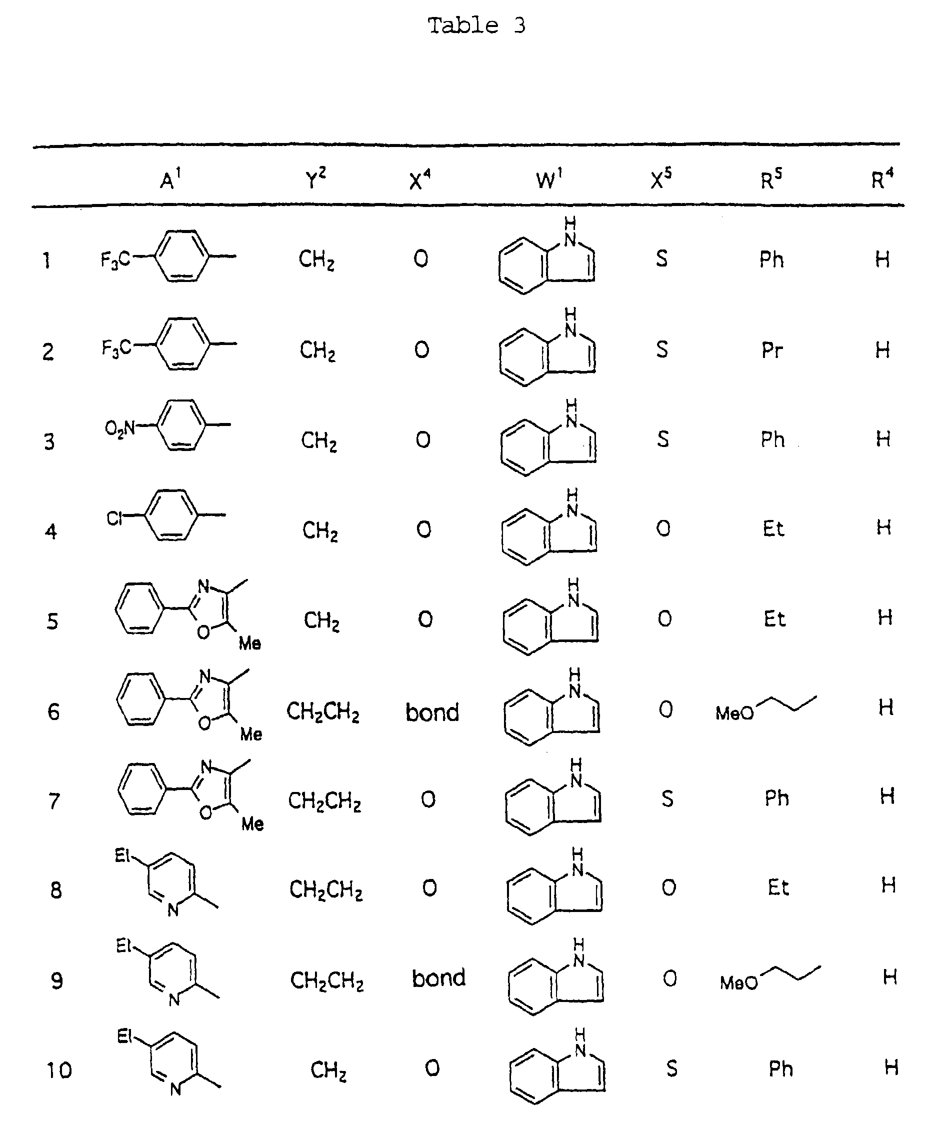

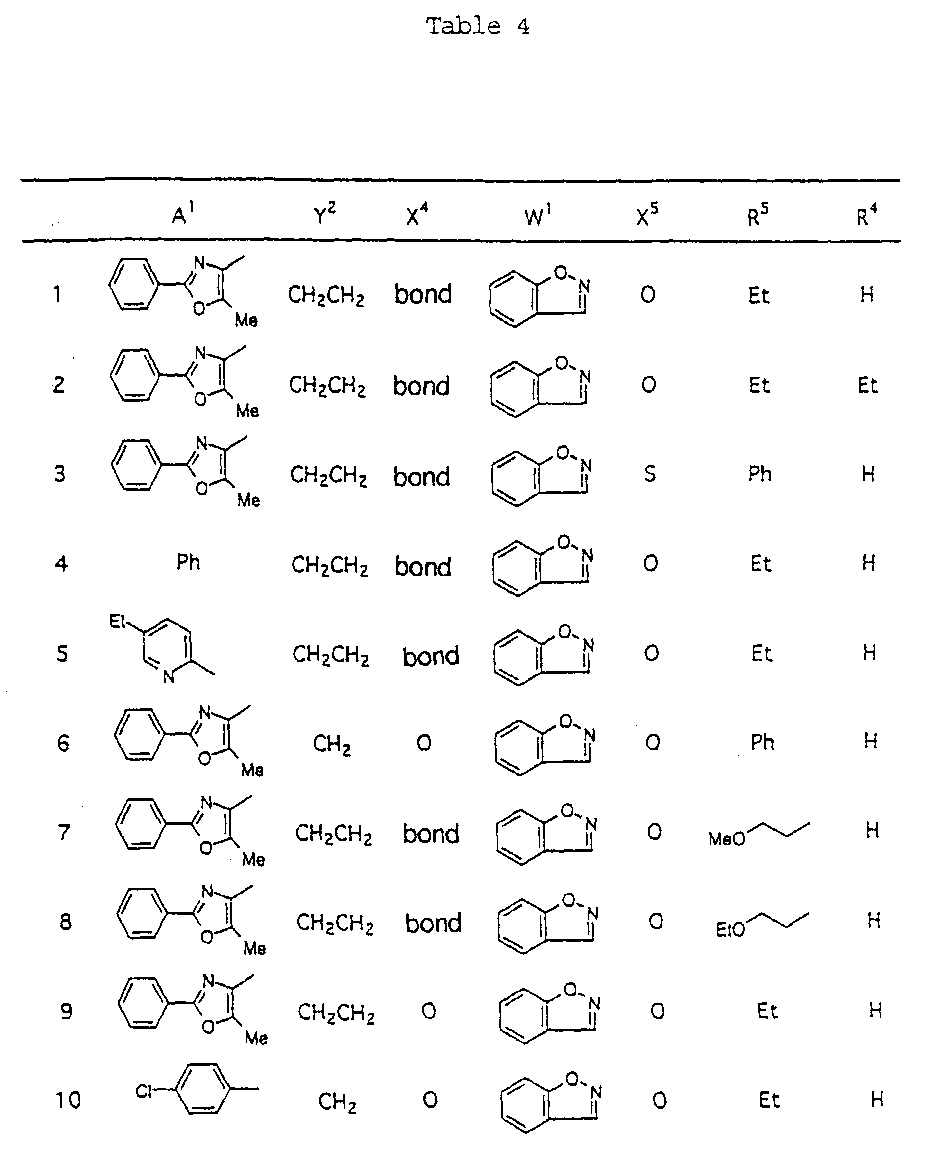

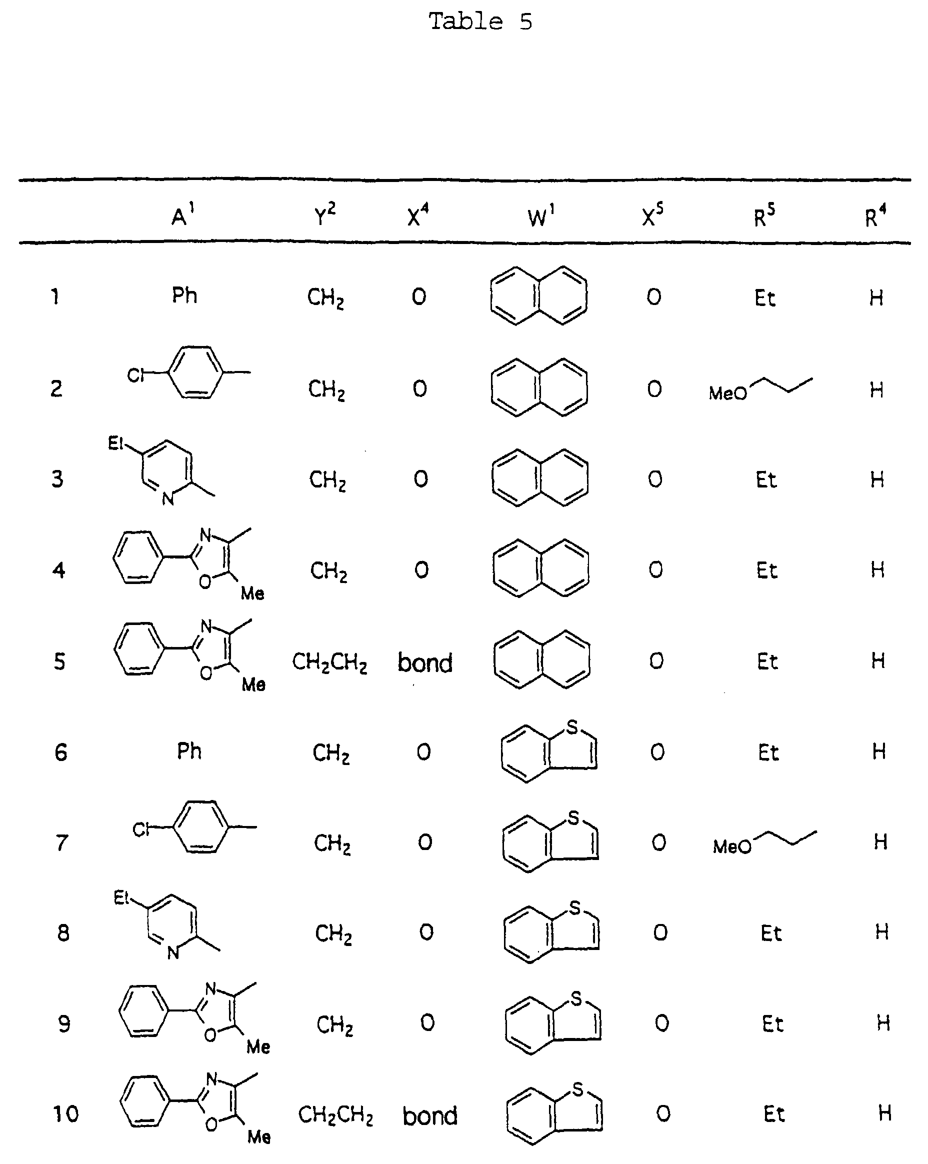

Representative examples of the compounds of the formula (I) other than the propionic acid esters of the formula (II), which are also according to the invention, are set forth in Tables 6 to 9.

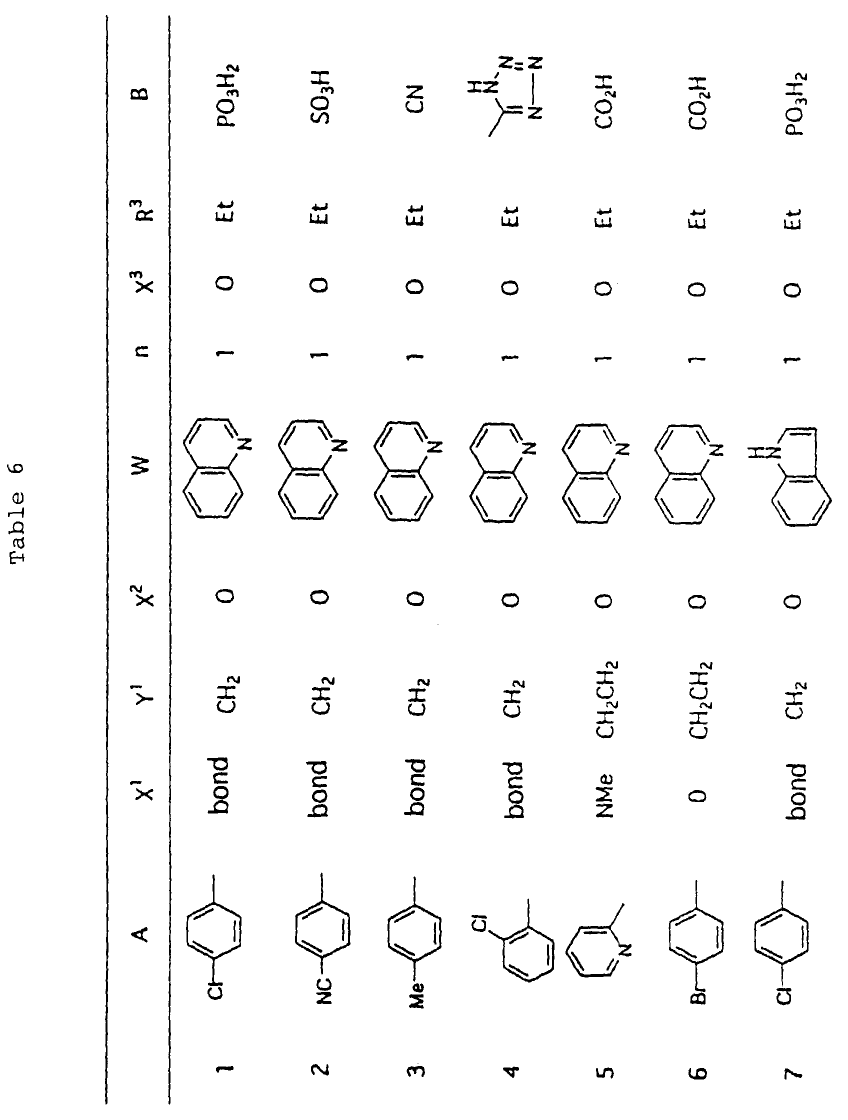

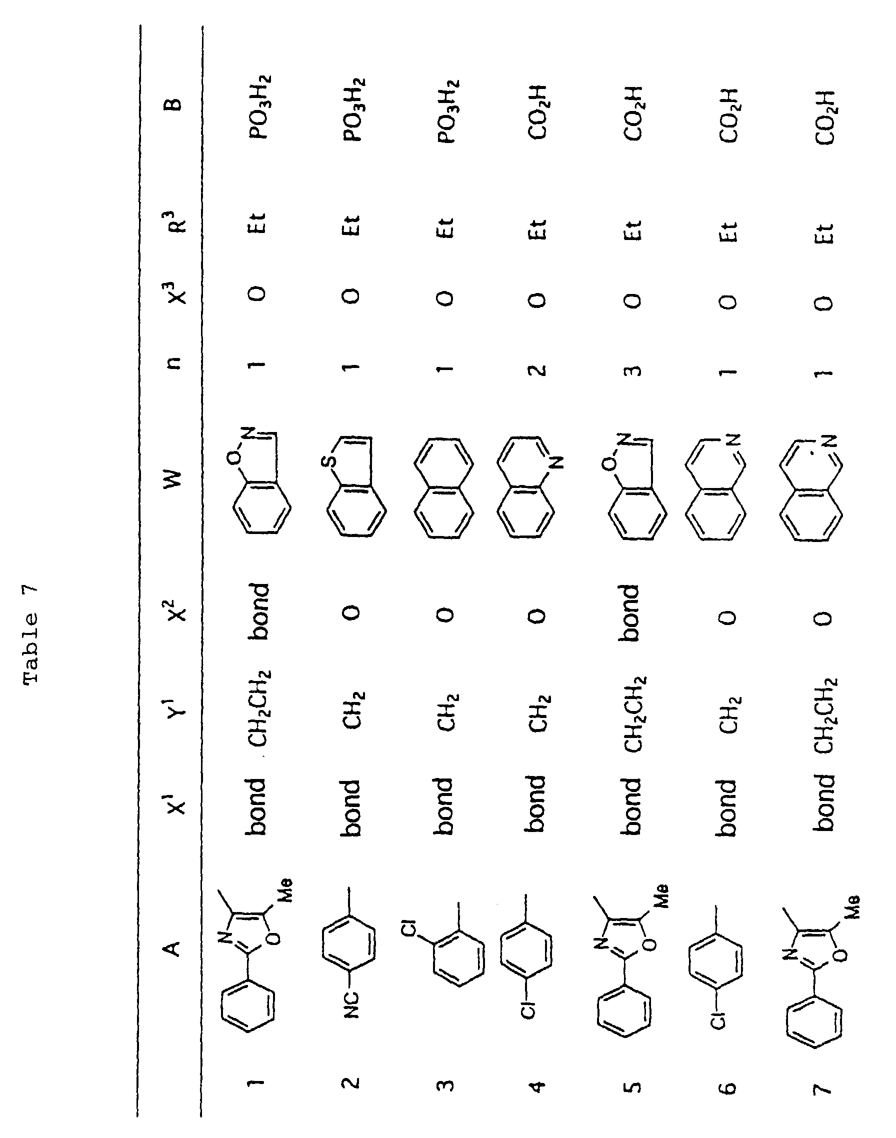

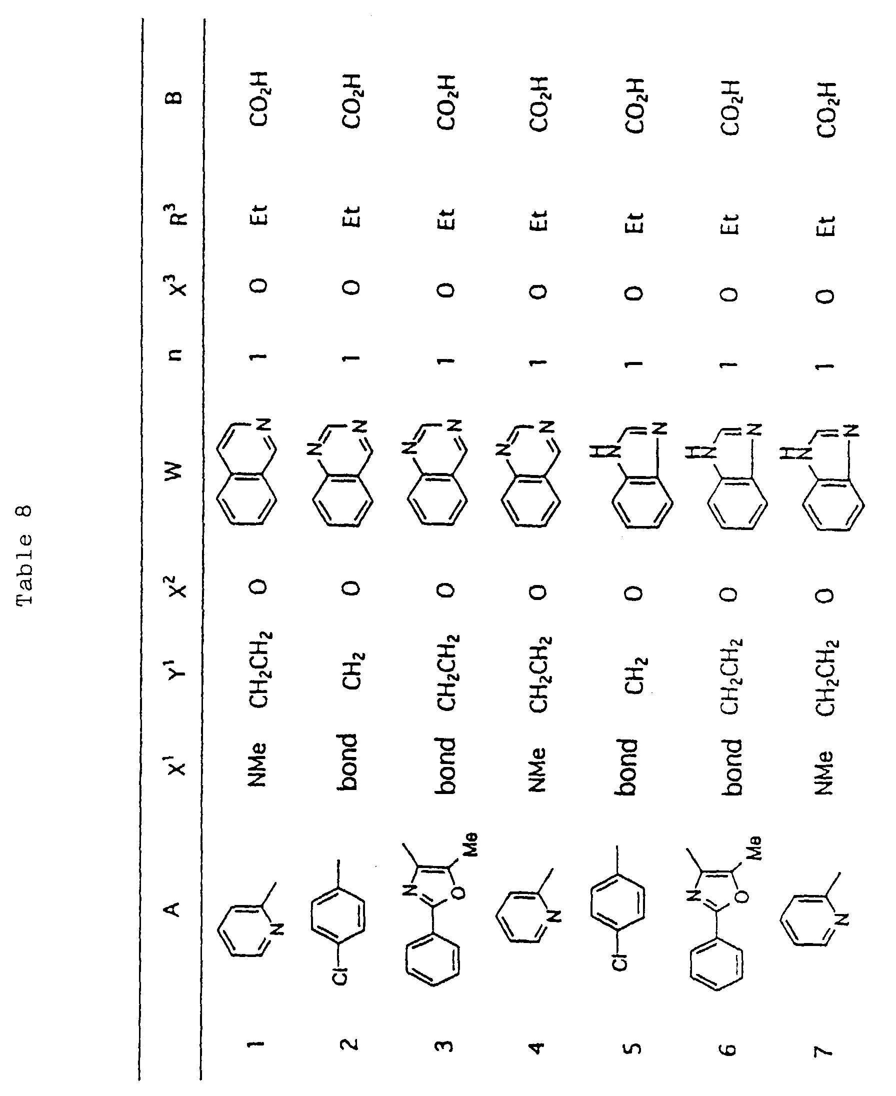

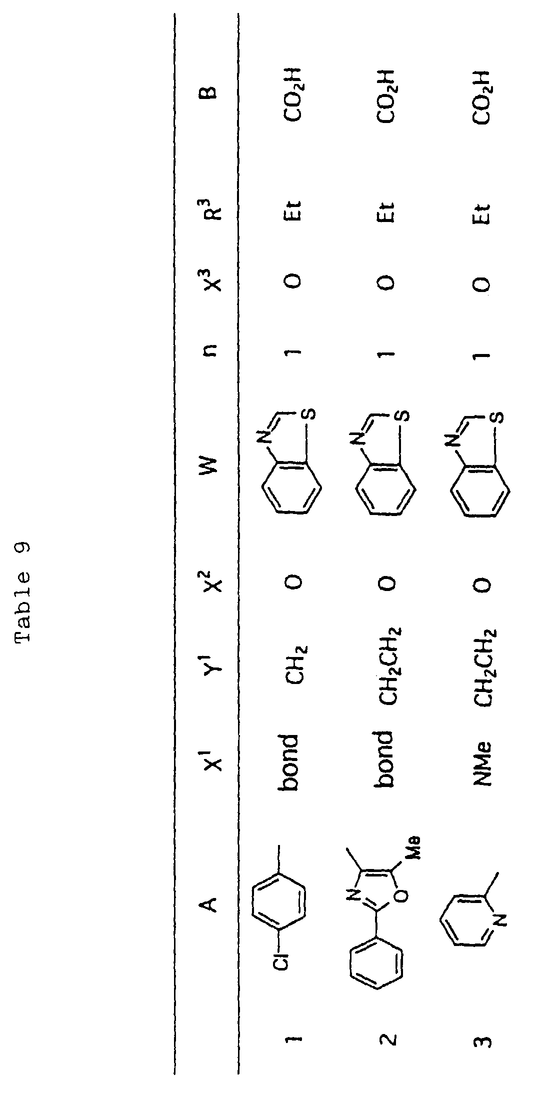

Pharmacological test data on the hypoglycemic effect and the hypolipidemic effect of the compounds of the invention are described below.

### Pharmacological Test

The hypoglycemic effect and hypolipidemic effect were examined using KKA^{y} mouse which was known as insulinindependent diabetic animal. A group of KKA^{y} mice (9 to 11 week age) were divided into several groups based on plasma glucose concentration. Each group included mice having an equal plasma glucose concentration. To the mouse was orally administered a compound of the invention in the form of suspension in a 1% methylcellulose solution. The administration was performed once a day and for a period of three days. To the control (mouse having been administered no hypoglycemic agent) was orally administered a 1% methylcellulose solution in the same manner.

After 18 hours from the final administration, blood was collected, and the plasma glucose concentration and the triglyceride concentration were measured. The measurements were performed using commercially available measuring kits (Glucose CII-TestWako and Triglyceride G-TestWako, respectively, both available from Wako Chemicals Co., Ltd.).

The plasma glucose concentration and the triglyceride concentration were examined for each of the divided mouse groups, and their ratio (%) per the corresponding values obtained in the control was calculated. The results are set forth in Table 10.

The same tests were also performed using the comparison compounds, that is, troglitazone and pioglitazone hydrochloride, which were known as pharmaceuticals for treating diabetes.

**Table 10**

| Compound | Dosage (mg/kg) | Plasma glucose concentration (% per control) | Triglyceride concentration (% per control) |
|---|---|---|---|
| Compound 1 | 100 | 68 | 60 |
| Compound 2 | 30 | 53 | 17 |
| Compound 3 | 30 | 63 | 66 |
| Compound 4 | 30 | 58 | 61 |
| Compound 5 | 30 | 77 | 81 |
| Compound 6 | 100 | 46 | 31 |
| Compound 7 | 1 | 76 | 87 |
| | 3 | 61 | 65 |
| | 10 | 60 | 45 |
| Troglitazone | 30 | 84 | 99 |
| | 100 | 68 | 101 |
| Pioglitazone hydrochloride | 30 | 68 | 84 |
| | 100 | 65 | 69 |

| | | | |
|---|---|---|---|
| Compound 1: 3-[6-[(4-trifluoromethyl)benzyloxy]-3-quinolyl]-2-(phenylthio)propionic acid | | | |
| Compound 2: 2-(2-methoxyethoxy)-3-[5-[(4-trifluoromethyl)benzyloxy]-2-indolyl]propionic acid | | | |
| Compound 3: 3-[6-(4-chlorobenzyloxy)-3-quinolyl]-2-ethoxypropionic acid | | | |
| Compound 4: 3-[7-(4-chlorobenzyloxy)-3-quinolyl]-2-ethoxypropionic acid | | | |
| Compound 5: 3-[7-(4-chlorobenzyloxy)-3-quinolyl]-2-phenoxypropionic acid | | | |
| Compound 6: 2-phenylthio-3-[3-[2-(2-phenyl-5-methyl-4-oxazolyl)ethyl]-1,2-benzisoxazol-6-yl]- propionic acid | | | |
| Compound 7: 2-ethoxy-3-[3-[2-(2-phenyl-5-methyl-4-oxazolyl)ethyl]-1,2-benzisoxazol-6-yl]- propionic acid | | | |

The results shown in Table 10 indicate that the compounds at invention significantly decrease glucose concentration and triglyceride concentration, as compared with the control group and therefore have excellent hypoglycemic activity as well as hypolipidemic activity.

The compounds of the invention can be administered either orally or parenterally. The orally administrable preparations may be in the form of pellets, capsules, powders, granules, and syrups. For the parenteral administration, application to mucosa using ophthalmic solutions, inhalations, sprays, or suppositories, application to body surface using ointments, intravenous or subcutaneous administration using injections can be utilized. The oral administrable preparations can be prepared using a conventionally employed excipient, disintegrator, binder, lubricant, dye, diluent, or the like. The excipient may be glucose or lactose. The disintegrator may be starch or carboxymethylcellulose calcium. The lubricant may be magnesium stearate or talc. The binder may be hydroxypropylcellulose, gelatin, or polyvinylpyrrolidone. The parenterally administrable injection may be prepared using distilled water for injection, physiological saline, or Ringer's solution.

The dosage of the compound of the invention for adult generally is approximately 0.1 to 200 mg/day when it is administered in the form of an injection, and approximately 1 to 2,000 mg/day when it is orally administered. The dosage can be adjusted depending on age, race, and clinical conditions.

In summary, the compounds of the invention show excellent plasma glucose concentration-decreasing activity as well as triglyceride concentration-decreasing activity, and therefore are of great value as a remedy for treatment of diabetes as well as a remedy for treatment of hyperlipemia.

The present invention is further described by the following examples, but the examples should not be construed to limit the invention.

### Example 1

### 3-[7-(4-Chlorobenzyloxy)-3-quinolyl]-2-(phenylthio)-propionic acid

### (1) methyl 3-[7-(4-chlorobenzyloxy)-3-quinolyl]-2-(phenylthio)propionate

To dimethylformamide (DMF, 6 mL) was added 60% sodium hydride (80 mg, 2.0 mmol.) under chilling with ice. To the mixture was dropwise added thiophenol (0.23 mL, 2.2 mmol.) for a period of one minute. The mixture was then allowed to reach room temperature. To the mixture was added a DMF solution (2 mL) of methyl 2-chloro-3-[7-(4-chlorobenzyloxy)-3-quinolyl]propionate (780 mg, 2.0 mmol.). The resulting mixture was stirred at room temperature for 30 minutes and then heated under reflux for 2 hours. The reaction mixture was poured into water, and extracted with ethyl acetate. The ethyl acetate portion was washed with water and dried over anhydrous sodium sulfate. The dried ethyl acetate portion was treated for distilling the solvent off. The residue was purified by silica gel column chromatography (hexane/ethyl acetate = 2/1) to give the desired compound as colorless oil (740 mg, yield 80%).
¹H-NMR (CDCl₃) δ:
   3.20 (1H, dd, J=6, 14Hz), 3.34 (1H, dd, J=8, 14 Hz), 3.60 (3H, s), 3.94 (1H, dd, J=6, 8Hz), 5.17 (2H, s), 7.2-7.5 (11H, m), 7.66 (1H, d, J=9Hz), 7.88 (1H, d, J=2Hz), 8.67 (1H, d, J=2Hz).

### (2) 3-[7-(4-chlorobenzyloxy)-3-quinolyl]-2-(phenylthio)-propionic acid

In ethanol (10 mL) was dissolved methyl 3-[7-(4-chlorobenzyloxy)-3-quinolyl]-2-(phenylthio)propionate (730 mg, 1.57 mmol.). To the resulting solution was added aqueous 1N sodium hydroxide solution (4 mL), and the mixture was stirred at room temperature for 3 hours. The mixture was then neutralized by addition of 1N hydrochloric acid. The precipitated crystalline product was collected by filtration, and washed with water and ethanol. The washed product was placed overnight under reduced pressure at room temperature to dryness. Thus, there was obtained the desired compound as a white crystalline product (470 mg, yield 66%). m.p. 157-158°C
¹H-NMR (DMSO-d₆) δ:
   3.12 (1H, dd, J=7, 14Hz), 3.23 (1H, dd, J=7, 14Hz), 4.17 (1H, t, J=7Hz), 5.27 (2H, s), 7.2-7.5 (9H, m), 7.53 (2H, d, J=8Hz), 7.82 (1H, d, J=9Hz), 8.12 (1H, s), 8.71 (1H, s), 12.76 (1H, brs).
IR (KBr) cm⁻¹:
   1710, 1700, 1620, 1580, 1480, 1470, 1430, 1380, 1370, 1320, 1260, 1220, 1160, 1080, 1010, 990, 830, 800, 720.

### Example 2

The following compound was prepared in the manner similar to that of Example 1.

### 3-[7-(4-Chlorobenzyloxy)-3-quinolyl]-2-(propylthio)-propionic acid

m.p. 50 - 60°C
¹H-NMR (CDCl₃) δ:
   1.05 (3H, t, J=7Hz), 1.6-1.8 (2H, m), 2.7-2.9 (2H, m), 3.22 (1H, dd, J=4, 14Hz), 3.45 (1H, dd, J=10, 14Hz), 3.58 (1H, dd, J=4, 10Hz), 5.12 (2H, s), 7.24 (1H, dd, J=2, 9Hz), 7.3-7.5 (4H, m), 7.59 (1H, d, J=2Hz), 7.68 (1H, d, J=9Hz), 8.08 (1H, d, J=2Hz), 8.87 (1H, d, J=2Hz).
IR (KBr) cm⁻¹:
   2960, 2930, 1710, 1620, 1500, 1260, 1240, 1220, 1180, 1130, 1100, 1020, 820.

### Example 3

The following compound was prepared in the manner similar to that of Example 1.

### 3-[6-[(4-Trifluoromethyl)benzyloxy]-3-quinolyl]-2-(phenylthio)propionic acid

m.p. 127 - 131°C
¹H-NMR (CDCl₃) δ:
   3.35 (1H, dd, J=5, 14Hz), 3.40 (1H, dd, J=9, 14Hz), 4.03 (1H, dd, J=5, 9Hz), 5.21 (2H, s), 7.08 (1H, d, J=3Hz), 7.29-7.42 (4H, m), 7.56-7.67 (6H, m), 8.00 (1H, d, J=2Hz), 8.03 (1H, d, J=9Hz), 8.74 (1H, d, J=2Hz).
IR (KBr) cm⁻¹:
   3458, 1718, 1622, 1585, 1506, 1438, 1419, 1389, 1329, 1236, 1169, 1124, 1066, 1018, 904, 827, 750, 692, 596.

### Example 4

### 2-Ethoxy-3-[6-(4-nitrobenzyloxy)-3-quinolyl]propionic acid

### (1) ethyl 2-ethoxy-3-[6-(methoxymethoxy)-3-quinolyl]-acrylate

In toluene (20 mL) were dissolved ethyl ethoxyacetate (3.92 g, 29.6 mmol.) and 6-(methoxymethoxy)quinoline-3-carbaldehyde (1.61 g, 7.41 mmol.). To the resulting solution was added potassium tert-butoxide (998 mg, 8.89 mmol.) under chilling with ice. The mixture was then stirred overnight. The reaction mixture was poured into water, and extracted with ethyl acetate. The organic portion was washed with water and saturated aqueous sodium chloride solution, and dried over anhydrous sodium sulfate. The dried portion was placed under reduced pressure to distill the solvent off. The residue was purified by silica gel flash column chromatography (hexane/ethyl acetate = 2/1 - 3/2) to give the desired compound (1.08 g, yield 44%).
¹H-NMR (CDCl₃) δ:
   1.40 (3H, t, J=7Hz), 1.41 (3H, t, J=7Hz), 3.54 (3H, s), 4.13 (2H, q, J=7Hz), 4.34 (2H, q, J=7Hz), 5.32 (2H, s), 7.06 (1H, s), 7.34 (1H, d, J=3Hz), 7.44 (1H, dd, J=3, 9Hz), 7.99 (1H, d, J=9Hz), 8.50 (1H, d, J=2Hz), 9.10 (1H, d, J=2Hz).

### (2) ethyl 2-ethoxy-3-[6-(methoxymethoxy)-3-quinolyl]-propionate

In ethanol (10 mL) was dissolved ethyl 2-ethoxy-3-[6-(methoxymethoxy)-3-quinolyl]acrylate (1.07 g, 3.23 mmol.). To the resulting solution was added 10% palladium/carbon (215 mg). The mixture was stirred for 20 hours in hydrogen stream. The catalyst was removed by filtration using a Celite, and the filtrate was concentrated. The residue was purified by silica gel flash column chromatography (hexane/ethyl acetate = 3/2) to give the desired compound (661 mg, yield 61%).
¹H-NMR (CDCl₃) δ:
   1.16 (3H, t, J=7Hz), 1.24 (3H, t, J=7Hz), 3.14 (1H, dd, J=8, 14Hz), 3.19 (1H, dd, J=5, 14Hz), 3.31-3.39 (1H, m), 3.53 (3H, s), 3.61-3.69 (1H, m), 4.07 (1H, dd, J=5, 8Hz), 4.20 (2H, q, J=7Hz), 5.30 (2H, s), 7.31 (1H, d, J=2Hz), 7.40 (1H, dd, J=2, 9Hz), 7.92 (1H, d, J=2Hz), 7.99 (1H, d, J=9Hz), 8.70(1H, d, J=2Hz).

### (3) ethyl 2-ethoxy-3-[6-(4-nitrobenzyloxy)-3-quinolyl]-propionate

In acetic acid (5 mL) was dissolved ethyl 2-ethoxy-3-[6-(methoxymethoxy)-3-quinolyl]propionate (379 mg, 1.14 mmol.), and to the solution were added water (5 mL) and concentrated sulfuric acid (2 drops). The mixture was then stirred at 90°C for 16 hours. The reaction mixture was poured into saturated aqueous sodium hydrogencarbonate solution and subsequently extracted with chloroform. The extract was dried over anhydrous sodium sulfate, filtered, and concentrated to give a crude ethyl 2-ethoxy-3-(6-hydroxy-3-quinolyl)propionate (300 mg).

The crude product (300 mg) was dissolved in N,N-dimethylformamide (3 mL), and to the solution was added sodium hydride (50 mg, 1.24 mmol.) under chilling with ice. The mixture was then stirred for 30 minutes. To the mixture was added 4-nitrobenzyl bromide (269 mg, 1.24 mmol.). The resulting mixture was allowed to reach room temperature and then stirred for 2 hours.

The reaction mixture was poured into water, and extracted with chloroform. The chloroform portion was washed with water and saturated aqueous sodium chloride solution, and dried over anhydrous sodium sulfate. The dried chloroform portion was placed under reduced pressure to distill chloroform off. The residue was purified by silica gel flash column chromatography (hexane/ethyl acetate = 3/2 - 1/1) to give the desired compound (232 mg, yield 48%).
¹H-NMR (CDCl₃) δ:
   1.15 (3H, t, J=7Hz), 1.23 (3H, t, J=7Hz), 3.15 (1H, dd, J=8, 14Hz), 3.20 (1H, dd, J=5, 14Hz), 3.31-3.38 (1H, m), 3.62-3.68 (1H, m), 4.07 (1H, dd, J=5, 8Hz), 4.19 (2H, q, J=7Hz), 5.30 (2H, s), 7.08 (1H, d, J=2Hz), 7.43 (1H, dd, J=2, 9Hz), 7.67 (2H, d, J=9Hz), 7.90 (1H, d, J=2Hz), 8.02 (1H, d, J=9Hz), 8.28 (2H, d, J=9Hz), 8.71 (1H, d, J=2Hz).

### (4) 2-ethoxy-3-[6-(4-nitrobenzyloxy)-3-quinolyl]propionic acid

In ethanol (3.75 mL) was dissolved ethyl 2-ethoxy-3-[6-(4-nitrobenzyloxy)-3-quinolyl]propionate (227 mg, 0.535 mmol.), and the resulting solution was stirred for 2.5 hours after addition of 1N lithium hydroxide (1.25 mL). The reaction mixture was made to pH 5 by slowly adding acetic acid under chilling with ice, and subsequently stirred for 30 minutes. The reaction mixture was poured into saturated aqueous sodium hydrogencarbonate solution and extracted with chloroform. The chloroform portion was dried over anhydrous sodium sulfate and placed under reduced pressure to distill chloroform off. The residue was recrystallized from ethyl acetate-hexane to give the desired compound (150 mg, yield 71%). m.p. 169 - 174°C.
¹H-NMR (CDCl₃) δ:
   1.26 (3H, t, J=7Hz), 3.28 (1H, dd, J=5, 14Hz), 3.35 (1H, dd, J=5, 14Hz), 3.54-3.61 (1H, m), 3.74-3.81 (1H, m), 4.25 (1H, t, J=5Hz), 5.26 (2H, s), 7.09 (1H, d, J=2Hz), 7.39 (1H, dd, J=2, 9Hz), 7.63 (2H, d, J=9Hz), 8.02 (1H, d, J=9Hz), 8.04 (1H, d, J=2Hz), 8.26 (2H, d, J=9Hz), 8.69 (1H, d, J=2Hz).
IR (KBr) cm⁻¹:
   3437, 3429, 1718, 1624, 1605, 1518, 1346, 1238, 1122, 1049, 839, 735.

### Example 5

The following compound was prepared in the manner similar to that of Example 4.

### 2-(2-Methoxyethoxy)-3-[5-[(4-trifluoromethyl)benzyloxy]-2-indolyl]propionic acid

m.p. 88 - 89°C
¹H-NMR (CD₃OD) δ:
   3.2-3.4 (2H, m), 3.52 (3H, s), 3.6-3.7 (2H, m), 3.8-3.9 (2H, m), 4.18 (1H, dd, J=3, 8Hz), 5.15 (2H, s), 6.23 (1H, s), 6.86 (1H, dd, J=2, 8Hz), 7.06 (1H, d, J=2Hz), 7.20 (1H, d, J=8Hz), 7.58 (2H, d, J=8Hz), 7.63 (2H, d, J=8Hz), 9.01 (1H, s).
IR (KBr) cm⁻¹:
   3400, 1710, 1620, 1590, 1480, 1450, 1415, 1370, 1325, 1290, 1230, 1180, 1160, 1120, 1090, 1060, 1040, 1015, 980, 960, 840, 820, 800.

### Example 6

### 3-[7-(4-Chlorobenzyloxy)-3-quinolyl]-2-(2-methoxyethoxy)propionic acid

m.p. 110 - 113°C
¹H-NMR (CDCl₃) δ:
   3.2-3.4 (2H, m), 3.40 (3H, s), 3.5-3.6 (2H, m), 3.7-3.8 (2H, m), 4.25 (1H, dd, J=5, 7Hz), 5.15 (2H, s), 7.35-7.45 (5H, m), 7.48 (1H, d, J=2Hz), 7.70 (1H, d, J=9Hz), 8.07 (1H, d, J=2Hz), 8.77 (1H, d, J=2Hz).
IR (KBr) cm⁻¹:
   3400, 1715, 1620, 1580, 1490, 1380, 1330, 1260, 1215, 1200, 1090, 1030, 1010, 965, 900, 845, 810, 780, 750.

### Example 7

### 2-(2-Methoxyethoxy)-3-[6-(4-nitrobenzyloxy)-3-quinolyl]propionic acid

m.p. 146 - 148°C
¹H-NMR (CDCl₃) δ:
   3.28 (1H, dd, J=6, 14Hz), 3.35-3.39 (1H, m), 3.39 (3H, s), 3.51-3.78 (4H, m), 4.27 (1H, dd, J=5, 6Hz), 5.29 (2H, s), 7.09 (1H, d, J=3Hz), 7.42 (1H, dd, J=3, 9Hz), 7.65 (2H, d, J=9Hz), 8.01 (1H, d, J=2Hz), 8.03 (1H, d, J=9Hz), 8.27 (2H, d, J=9Hz), 8.71 (1H, d, J=2Hz).
IR (KBr) cm⁻¹:
   3435, 2922, 1720, 1624, 1606, 1521, 1344, 1236, 1174, 1109, 1043, 841, 737.

### Example 8

### 3-[6-(4-Chlorobenzyloxy)-3-quinolyl)-2-ethoxypropionic acid

m.p. >250°C (decomp.)
¹H-NMR (DMSO-d₆:CD₃OD=10:1 v/v) δ:
   1.02 (3H, t, J=7Hz), 2.95 (1H, dd, J=8, 14Hz), 3.10 (1H, dd, J=4, 14Hz), 3.17-3.24 (1H, m), 3.56-3.62 (1H, m), 3.80 (1H, dd, J=4, 8Hz), 5.23 (2H, s), 7.36 (1H, d, J=3Hz), 7.38 (1H, dd, J=3, 9Hz), 7.46 (2H, d, J=8Hz), 7.54 (2H, d, J=8Hz), 7.88 (1H, d, J=9Hz), 8.00 (1H, d, J=2Hz), 8.62 (1H, d, J=2Hz).
IR (KBr) cm⁻¹:
   3425, 2968, 1626, 1594, 1508, 1417, 1387, 1344, 1240, 1217, 1124, 1107, 1092, 1055, 1014, 831, 806, 756, 703.

### Example 9

### 3-[7-(4-Chlorobenzyloxy)-3-quinolyl]-2-ethoxypropionic acid

m.p. 75 - 78°C
¹H-NMR (CDCl₃) δ:
   1.27 (3H, t, J=7Hz), 3.2-3.4 (2H, m), 3.55-3.65 (1H, m), 3.75-3.85 (1H, m), 4.2-4.3 (1H, m), 5.12 (2H, s), 7.2-7.3 (1H, m), 7.37 (2H, d, J=9Hz), 7.42 (2H, d, J=9Hz), 7.53 (1H, d, J=2Hz), 7.70 (1H, d, J=9Hz), 8.12 (1H, s), 8.76 (1H, d, J=2Hz).
IR (KBr) cm⁻¹:
   3425, 2975, 2875, 1720, 1620, 1490, 1430, 1410, 1385, 1330, 1260, 1220, 1170, 1125, 1090, 1040, 1010, 900, 845, 810, 770, 665.

### Example 10

### 3-[7-(4-Chlorobenzyloxy)-3-quinolyl]-2-phenoxypropionic acid

m.p. 192 - 194°C
¹H-NMR (DMSO-d₆) δ:
   3.3-3.4 (2H, m), 4.95-5.05 (1H, m), 5.27 (2H, s), 6.8-7.25 (5H, m), 7.30 (1H, dd, J=2, 9Hz), 7.42 (1H, d, J=2Hz), 7.46 (2H, d, J=8Hz), 7.54 (2H, d, J=8Hz), 7.85 (1H, d, J=9Hz), 8.19 (1H, s), 8.79 (1H, d, J=2Hz).
IR (KBr) cm⁻¹:
   3425, 1720, 1625, 1600, 1585, 1490, 1430, 1410, 1380, 1330, 1300, 1260, 1230, 1170, 1150, 1130, 1090, 1010, 990, 920, 885, 830, 810, 750, 690, 670, 540.

### Example 11

### 3-[5-[(4-Trifluoromethyl)benzyloxy]-2-indolyl]-2-phenoxypropionic acid

¹H-NMR (CDCl₃) δ:
   3.40-3.45 (2H, m), 4.95-5.00 (1H, m), 5.13 (2H, s), 6.28 (1H, s), 6.86 (1H, dd, J=2, 9Hz), 7.0-7.4 (6H, m), 7.21 (1H, d, J=9Hz), 7.56 (2H, d, J=8Hz), 7.62 (2H, d, J=8Hz), 8.33 (1H, brs).
IR (KBr) cm⁻¹:
   3475, 1720, 1625, 1590, 1490, 1455, 1420, 1325, 1295, 1230, 1180, 1175, 1110, 1090, 1070, 1020, 870, 840, 830, 810, 800, 790, 760, 690.

### Example 12

### 2-Ethoxy-3-[3-[2-(2-phenyl-5-methyl-4-oxazolyl)-ethyl]-1,2-benzisoxazol-6-yl]propionic acid

### (1) 6-bromo-3-[2-(2-phenyl-5-methyl-4-oxazolyl)ethyl]-1,2-benzisoxazole

In a mixture of methanol and acetone (2:5, 5 mL) was dissolved 6-amino-3-[2-(2-phenyl-5-methyl-4-oxazolyl)-ethyl]-1,2-benzisoxazole (400 mg, 1.25 mmol.). To the resulting solution was added 47% hydrobromic acid (0.94 g) for a period of 1 minute under chilling with ice so as to keep the solution temperature at 1°C. After 10 minutes, an aqueous sodium nitrite solution (110 mg, 1.58 mmol./0.2 mL) was dropwise added for a period of one minute, and the resulting mixture was stirred for 10 minutes under the same conditions. Subsequently, a solution of copper(I) bromide in 47% hydrobromic acid (110 mg/520 mg) was dropwise added for a period of one minute. The resulting mixture was warmed to 40°C (inner temperature), and then stirred for 30 minutes. After the reaction was complete, to the mixture were added ethyl acetate and saturated aqueous sodium hydrogencarbonate solution under chilling with ice. The organic portion was separated, washed with water, and dried over anhydrous sodium sulfate. The dried organic portion was treated to distill the solvent off. The residue was purified by silica gel column chromatography (hexane/ethyl acetate = 3/1) to give 640 mg (yield 89%) of the desired compound as a white crystalline product.
¹H-NMR (CDCl₃) δ:
   2.17 (3H, s), 3.04 (2H, t, J=7Hz), 3.36 (2H, t, J=7Hz), 7.35 (1H, dd, J=2, 9Hz), 7.4-7.5 (4H, m), 7.73 (1H, d, J=2Hz), 7.95-8.0 (2H, m).

### (2) 3-[2-(2-phenyl-5-methyl-4-oxazolyl)ethyl]-1,2-benzisoxazol-6-carbaldehyde

In dry tetrahydrofuran (10 mL) was dissolved 6-bromo-3-[2-(2-phenyl-5-methyl-4-oxazolyl)ethyl]-1,2-benzisoxazole (380 mg, 1.00 mmol.). To the resulting solution was dropwise added slowly n-butyl lithium (2.5 M) (1.5 mL, 3.80 mmol.) at -78°C for a period of 5 minutes in a nitrogen gas atmosphere. After 30 minutes, a tetrahydrofuran solution of dry DMF (366 mg, 5.0 mmol./1 mL) was added for a period of 3 minutes, and the resulting mixture was stirred for 30 minutes under the same conditions. After disappearance of the starting compound was confirmed, the reaction mixture was allowed to reach room temperature. To the mixture were then added saturated aqueous ammonium chloride solution and ethyl acetate. The organic portion was separated, washed with water, dried over anhydrous sodium sulfate, and treated to distill the solvent off. The residue was purified by silica gel column chromatography (hexane/ethyl acetate = 2/1) to give 125 mg (yield 38%) of the desired compound as colorless oil.
¹H-NMR (CDCl₃) δ:
   2.16 (3H, s), 3.07 (2H, t, J=7Hz), 3.43 (2H, t, J=7Hz), 7.4-7.5 (3H, m), 7.71 (1H, d, J=8Hz), 7.78 (1H, dd, J=8Hz), 7.9-8.0 (2H, m), 8.03 (1H, s), 10.14 (1H, s).

### (3) 6-(2-methoxyvinyl)-3-[2-(2-phenyl-5-methyl-4-oxazolyl)ethyl]-1,2-benzisoxazole

In dry tetrahydrofuran (6 mL) was dissolved (methoxymethyl)phosphonium chloride (598 mg, 1.74 mmol.). To the resulting solution was added lithium diisopropylamide (2.0 M) (0.87 mL, 1.74 mmol.) at -15°C for a period of one minute in a nitrogen gas atmosphere. The mixture was stirred for 40 minutes under the same conditions, and to the mixture was added a solution of 3-[2-(2-phenyl-5-methyl-4-oxazolyl) ethyl]-1,2-benzisoxazol-6-carbaldehyde in tetrahydrofuran (290 mg, 0.87 mmol./2 mL) for a period of one minute. The mixture was allowed to reach room temperature, and stirred for 30 minutes. To the mixture were then added saturated aqueous ammonium chloride solution and ethyl acetate. The organic portion was separated, washed with water, dried over anhydrous sodium sulfate, and treated to distill the solvent off. The residue was purified by silica gel column chromatography (hexane/ethyl acetate = 3/1) to give 68 mg (yield 22%) of the desired compound (mixture of E isomer and Z isomer) as colorless oil.

### (4) 6-(2,2-diethoxyethyl)-3-[2-(2-phenyl-5-methyl-4-oxazolyl)ethyl]-1,2-benzisoxazole

In ethanol (4 mL) were dissolved 6-(2-methoxyvinyl)-3-[2-(2-phenyl-5-methyl-4-oxazolyl)ethyl]-1,2-benzisoxazole (65 mg, 0.18 mmol.) and p-toluenesulfonic acid hydrate (4 mg). The solution was heated to 90°C and refluxed overnight under heating. After the reaction was complete, the mixture was treated to distill ethanol off. The residue was mixed with ethyl acetate and saturated aqueous sodium hydrogencarbonate solution, and then the organic portion was separated. The organic portion was washed with water, dried over anhydrous sodium sulfate, and treated to distill the solvent off, to give 70 mg (yield 92%) of the desired compound as colorless oil.
¹H-NMR (CDCl₃) δ:
   1.16 (3H, t, J=7Hz), 2.15 (3H, s), 3.0-3.1 (4H, m), 3.35 (2H, t, J=7Hz), 3.4-3.5 (2H, m), 3.6-3.75 (2H, m), 4.65 (1H, t, J=6Hz), 7.14 (1H, dd, J=2, 9Hz), 7.4-7.5 (5H, m), 7.9-8.1 (2H, m).

### (5) 2-ethoxy-3-[3-[2-(2-phenyl-5-methyl-4-oxazolyl)-ethyl]-1,2-benzisoxazol-6-yl]propionitrile

In dry dichloromethane (2 mL) was dissolved 6-(2,2-diethoxyethyl)-3-[2-(2-phenyl-5-methyl-4-oxazolyl)ethyl]-1,2-benzisoxazole (67 mg, 0.16 mmol.). To the resulting solution was dropwise added trimethylsilylcyanide (0.08 mL) for a period of one minute. Subsequently, boron trifluoride-ether complex (5 µL, 0.05 mmol.) was added and stirred at room temperature for 30 minutes. After one hour and two hours, boron trifluoride-ether complex (5 µL) was added, and the mixture was stirred overnight at room temperature. After completion of the reaction was confirmed, to the reaction mixture were added chloroform and saturated aqueous sodium hydrogencarbonate solution. The organic portion was separated, washed with water, dried over anhydrous sodium sulfate, and treated to distill the solvent off. The residue was purified by column chromatography (hexane/ethyl acetate = 4/1) to give 34 mg (yield 53%) of the desired compound as colorless oil.
¹H-NMR (CDCl₃) δ:
   1.23 (3H, t, J=7Hz), 2.17 (3H, s), 3.05 (2H, t, J=7Hz), 3.2-3.3 (2H, m), 3.37 (2H, t, J=7Hz), 3.4-3.6 (1H, m), 3.8-3.9 (1H, m), 4.32 (1H, t, J=7Hz), 7.16 (1H, d, J=8Hz), 7.4-7.5 (4H, m), 7.53 (1H, d, J=8Hz), 7.9-8.0 (2H, m).

### (6) 2-ethoxy-3-[3-[2-(2-phenyl-5-methyl-4-oxazolyl)-ethyl]-1,2-benzisoxazol-6-yl]propionic acid

In ethanol (1.5 mL) was dissolved 2-ethoxy-3-[3-[2-(2-phenyl-5-methyl-4-oxazolyl)ethyl]-1,2-benzisoxazol-6-yl]propionitrile (34 mg, 0.085 mmol.). To the resulting solution was slowly added 6N aqueous sodium hydroxide solution (0.50 mL). The mixture was heated to 90°C, and refluxed for 90 minutes under heating. After disappearance of the starting compound was confirmed, the mixture was chilled with ice, made to pH 4 - 5 by slowly adding concentrated hydrochloric acid, and allowed to stand overnight. The precipitated crystalline product was collected by filtration and washed with 30 mL of water. The washed product was placed under reduced pressure at 50°C for 3 hours, to dryness. There was obtained 19 mg (yield 56%) of the desired compound as a white crystal-line product.
¹H-NMR (DMSO-d₆) δ:
   1.01 (3H, t, J=7Hz), 2.16 (3H, s), 2.99 (2H, t, J=7Hz), 3.0-3.2 (2H, m), 3.3-3.5 (3H, m), 3.37 (2H, m), 3.5-3.6 (1H, m), 4.07 (1H, dd, J=4, 8Hz), 7.24 (1H, d, J=8Hz), 7.4-7.6 (4H, m), 7.73 (1H, d, J=8Hz), 7.9-8.0 (2H, m), 12.7 (1H, brs).

### Example 13

### 2-Phenylthio-3-[3-[2-(2-phenyl-5-methyl-4-oxazolyl)-ethyl]-1,2-benzisoxazol-6-yl]propionic acid

### (1) 6-acetamido-3-[2-[2-phenyl-5-methyl-4-oxazolyl)-ethyl]-1,2-benzisoxazole

In 25 mL of dry THF was dissolved 6-acetamido-3-methyl-1,2-benzisoxazole (1.5 g, 7.89 mmol.). To the resulting solution was dropwise added 2M LDA (9.0 mL, 18.0 mmol.) at -78°C for a period of 10 minutes in a nitrogen gas atmosphere. The mixture was then stirred for 10 minutes under the same conditions. Subsequently, a solution of 4-iodomethyl-5-methyl-2-phenyloxazole in THF (1.9 g, 7.89 mmol./4 mL) was dropwise added for one minute, and the resulting mixture was stirred for 30 minutes under the same conditions. After the reaction was complete, the reaction mixture was allowed to reach room temperature, and mixed with saturated aqueous ammonium chloride solution and ethyl acetate. The ethyl acetate portion was separated, dried over anhydrous sodium sulfate, and filtered. The filtrate was treated to distill ethyl acetate off. The residue was purified by silica gel chromatography (chloroform - chloroform/methanol= 100/1) to give the desired compound (yield 32%) as a white crystalline product.
¹H-NMR (CDCl₃) δ:
   2.14 (3H, s), 2.19 (3H, s), 3.02 (2H, t, J=8Hz), 3.32 (2H, t, J=8Hz), 7.13 (1H, dd, J=2, 9Hz), 7.35-7.45 (4H, m), 7.83 (1H, br-s), 7.9-8.0 (2H, m), 8.08 (1H, d, J=2Hz).

### (2) 6-amino-3-[2-(2-phenyl-5-methyl-4-oxazolyl)ethyl]-1,2-benzisoxazole

In 1N hydrochloric acid (9 mL) was suspended 6-acetamido-3-[2-(2-phenyl-5-methyl-4-oxazolyl)ethyl]-1,2-benzisoxazole (700 mg, 1.9 mmol.) obtained in (1) above. The suspension was heated to 100°C and refluxed for 6 hours under heating. After the reaction was complete, the reaction mixture was allowed to reach room temperature. The mixture was then neutralized by addition of saturated aqueous sodium carbonate solution, and mixed with ethyl acetate. The ethyl acetate portion was then separated. The ethyl acetate portion was washed with water, dried over anhydrous sodium sulfate, and filtered. The filtrated was treated to distill ethyl acetate off, to give 530 mg (yield 81%) of the desired compound as colorless oil.
¹H-NMR (CDCl₃) δ:
   2.15 (3H, s), 3.00 (2H, t, J=7Hz), 3.27 (2H, t, J=7Hz), 4.01 (2H, br-s), 6.54 (1H, dd, J=2, 9Hz), 6.70 (1H, d, J=2Hz), 7.2-7.5 (4H, m), 8.0-8.2 (2H, m).

### (3) methyl 2-chloro-3-[3-[2-(2-phenyl-5-methyl-4-oxazolyl)ethyl]-1,2-benzisoxazol-6-yl]propionate

In a mixture of acetone (13 mL) and water (3 mL) was dissolved 6-amino-3-[2-(2-phenyl-5-methyl-4-oxazolyl)-ethyl]-1,2-benzisoxazole (1.06 g, 3.50 mmol.) prepared in (2) above. To the resulting solution was added concentrated hydrochloric acid (0.96 mL) under chilling with ice.

After 10 minutes, to the ice-chilled mixture was dropwise added aqueous sodium nitrite solution (360 mg, 4.20 mmol./0.5 mL) for a period of 5 minutes. The mixture was then stirred at the same temperature for 10 minutes, and to the mixture was dropwise added methyl acrylate (2.3 mL) for a period of one minute. After 10 minutes, cuprous oxide (50 mg) was slowly added to the mixture. The mixture was then warmed to 45°C (outer temperature), and stirred vigorously for 15 minutes at the same temperature. After the reaction was complete, to the mixture were added aqueous sodium hydrogencarbonate solution and ethyl acetate. The organic portion was separated, washed with water, dried over arhydrous sodium sulfate, and filtered. The filtrate was treated to distill ethyl acetate off. The residue was purified by column chromatography (n-hexane - ethyl acetate) to give 910 mg (yield 61%) of the desired compound as yellow oil.
¹H-NMR (CDCl₃) δ:
   2.16 (3H, s), 3.04 (2H, t, J=7Hz), 3.36 (2H, t, J=7Hz), 3.31 (1H, dd, J=7, 14Hz), 3.51 (1H, dd, J=7, 14Hz), 3.75 (3H, s), 4.49 (1H, t, J=7Hz), 7.11 (1H, d, J=9Hz), 7.4-7.5 (4H, m), 7.51 (1H, m), 7.98 (2H, dd, J=2, 9Hz).

### (4) Methyl 2-phenylthio-3-[3-[2-(2-phenyl-5-methyl-4-oxazolyl)ethyl]-1,2-benzisoxazol-6-yl]propionate

In DMF (4 mL) was slowly added sodium hydride (28 mg, 0.70 mmol.) under chilling with ice. To the mixture was added thiophenol (0.07 mL, 0.70 mmol.) for a period of one minute. After 30 minutes, the mixture was allowed to reach room temperature, and to the mixture was slowly added a solution of methyl 2-chloro-3-[3-[2-(2-phenyl-5-methyl-4-oxazolyl)ethyl]-1,2-benzisoxazol-6-yl]propionate (270 mg, 0.64 mmol) in DMF (2 mL). After 30 minutes, the mixture was heated to 80°C, and stirred under heating for 2 hours. After the reaction was complete, the reaction mixture was poured into water and then extracted with ethyl acetate. The ethyl acetate portion was washed with water, dried over anhydrous sodium sulfate, and filtered. The filtrate was treated to distill the solvent off. The residue was purified by silica gel column chromatography (hexane/ethyl acetate = 2/1) to give 210 mg (yield 68%) of the desired compound as colorless oil.
¹H-NMR (CDCl₃) δ:
   2.14 (3H, s), 3.03 (2H, t, J=7Hz), 3.18 (1H, dd, J=9, 14Hz), 3.25-3.4 (3H, m), 3.91 (1H, dd, J=7, 10Hz), 7.07 (1H, d, J=8Hz), 7.3-7.5 (10H, m), 7.9-8.0 (2H, m).

### (5) 2-phenylthio-3-[3-[2-(2-phenyl-5-methyl-4-oxazolyl)-ethyl]-1,2-benzisoxazol-6-yl]propionic acid

In 4 mL of ethanol was dissolved methyl 2-phenyl-thio-3-[3-[2-(2-phenyl-5-methyl-4-oxazolyl)ethyl]-1,2-benzisoxazol-6-yl]propionate (200 mg, 0.41 mmol.) prepared in (4) above, and to the resulting solution was added 1N NaOH (1 mL) at room temperature. The mixture was then stirred one hour. After the reaction was complete, the reaction mixture was neutralized by addition of 1N HCl, and then stirred overnight. The precipitated crystalline product was collected by filtration, washed with water, and placed under reduced pressure to dryness, to give 175 mg (yield 88%) of the desired compound as a white powder. m.p. 145 - 147°C.
¹H-NMR (DMSO-d₆) δ:
   2.16 (3H, s), 2.98 (2H, t, J=7Hz), 3.14 (1H, dd, J=7, 14Hz), 3.2-3.4 (3H, m), 4.14 (1H, t, J=7Hz), 7.3-7.7 (10H, m), 7.75 (1H, d, J=7Hz), 7.90 (2H, dd, J=2, 7 Hz), 12.7 (1H, brs).
IR (KBr) cm⁻¹:
   3050, 2900, 1720, 1700, 1650, 1620, 1540, 1480, 1440, 1430, 1420, 1410, 1340, 1220, 1180, 1170, 1130, 1020, 840, 820, 780, 740, 710, 680.

## Claims

1. A compound having the following formula (I) and its salt: 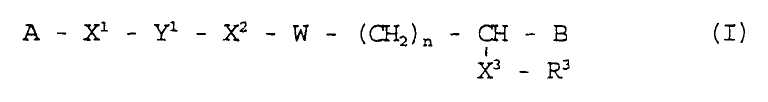 wherein A represents an aryl or heterocyclic group which may have a substituent; X¹ represents a single bond, an oxygen atom, a sulfur atom, or NR¹ in which R¹ is a hydrogen atom, an alkyl group having 1 to 8 carbon atoms, or an aralkyl group; Y¹ represents an alkylene chain having 1 to 8 carbon atoms and possibly having a substituent; X² represents a single bond, an oxygen atom, a sulfur atom, or NR² in which R² is a hydrogen atom, an alkyl group having 1 to 8 carbon atoms, or an aralkyl group; W represents a naphthalene ring which may have a substituent, or a bicyclic group comprising a benzene ring which is fused with a heterocycle which contains at least one nitrogen or sulfur atom as ring member atoms and possibly having a substituent; B represents a carboxyl group, a cyano group, an alkoxycarbonyl group having 2 to 9 carbon atoms, an aralkyloxycarbonyl group, an aryloxycarbonyl, a sulfonic acid group, a phosphonic acid group, or a tetrazolyl group; X³ represents an oxygen atom or a sulfur atom; R³ represents an alkyl group having 1 to 8 carbon atoms, an aralkyl group, or an aryl group, all of which may have a substituent; and n is an integer of 1 to 4.

2. A propionic acid derivative having the following formula (II) and its salt: 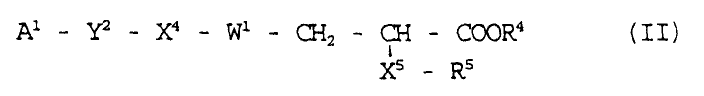 wherein A¹ represents an aryl or heterocyclic group which may have a substituent; Y² represents an alkylene chain having 1 to 5 carbon atoms; X⁴ represents a single bond, an oxygen atom, or a sulfur atom; W¹ represents a naphthalene ring, a quinoline ring, an indole ring, a benzisoxazole ring, or benzo[b]thiophene ring, all of which may have a substituent; R⁴ represents a hydrogen atom or an alkyl group having 1 to 8 carbon atoms; X⁵ represents an oxygen atom or a sulfur atom; R⁵ represents an alkyl group having 1 to 8 carbon atoms, an aralkyl group, or an aryl group, all of which may have a substituent.
